**Europäisches Patentamt**

**European Patent Office**

**Office européen des brevets**

(19)

(11) Numéro de publication: **0 147 274**
**A1**

## (12) DEMANDE DE BREVET EUROPEEN

(21) Numéro de dépôt: **84402445.5**

(22) Date de dépôt: **29.11.84**

(51) Int. Cl.⁴: **A 61 F 5/47**

(30) Priorité: **01.12.83 FR 8319195**
**07.06.84 FR 8408916**

(43) Date de publication de la demande:
**03.07.85 Bulletin 85/27**

(84) Etats contractants désignés:
**AT BE CH DE GB IT LI LU NL SE**

(71) Demandeur: **BIOMASYS Société dite:**
**Château de Beauregard Nan-Sous-Thil**
**F-21390 Precy-sous-Thil(FR)**

(72) Inventeur: **Lapeyre, Didier**
**Chaignes**
**F-27120 Pacy sur Eure(FR)**

(72) Inventeur: **Slonina, Jean-Pierre**
**23 bis, route de la Cascade**
**F-78110 Le Vesinet(FR)**

(74) Mandataire: **Ores, Irène et al,**
**CABINET ORES 6, Avenue de Messine**
**F-75008 Paris(FR)**

(54) **Perfectionnements apportés aux dispositifs intra-utérins.**

(57) Dispositifs intra-utérins en matière plastique souple, comportant une tige dont une extrêmité porte deux branches et l'autre un fil d'éjection.

La tige (1) et les branches (2, 3) sont revêtues d'une mince couche de carbone et les branches (2, 3) portent des protubérances (4, 5 ou 19, 20) réalisées en totalité ou en partie en carbone, qui sont soit moulées en même temps que l'ensemble (1, 2, 3), soit produites séparément et rapportées sur les extrêmités des branches (2, 3). En outre, des perles (8), avantageusement séparées par des rondelles (9) en matière plastique souple, sont constituées par ou contiennent une matière propre à se libérer progressivement dans la cavité utérine, tel que métal pur, cuivre notamment, poreux ou non, céramique, carbone poreux, dont les pores contiennent une ou plusieurs matières contraceptives et/ou thérapeutiques aptes à se libérer progressivement dans la cavité utérine.

Application à la contraception ou aux traitements thérapeutiques locaux.

FIG. 1a

- 1 -

La présente invention est relative à un nouveau dispositif intra-utérin, utilisable notamment en tant que contraceptif ou en tant que dispositif de libération de substances médicamenteuses de traitement ou de contraception.

L'on connait des dispositifs intra-utérins contraceptifs, ou stérilets, équipés de fils de cuivre disposés en spirale, qui libèrent les ions cuivre dans la cavité endo-utérine. Toutefois, de tels dispositifs contraceptifs présentent un certain nombre d'inconvénients parmi lesquels il y a lieu de citer la diminution de désorption des ions cuivre dans le temps, les fragmentations des fils consécutives à une attaque chimique hétérogène et les difficultés d'adaptation de la forme des stérilets aux dimensions de la cavité utérine, en raison de la rigidité des spirales de fil de cuivre dont sont constitués ces stérilets. Des améliorations ont été apportées à cette première génération de dispositifs intra-utérins ; de telles améliorations ont proposé de réaliser le dispositif intra-utérin sous la forme d'une tige en polyéthylène souple, à deux branches flexibles, réalisée par moulage par injection, dont les extrêmités des branches sont pourvues de demi-sphères et dont l'extrêmité libre de la tige est pourvue d'une boucle flexible ; autour de la tige est enroulé un fil de cuivre dont la surface est renforcée par un noyau d'argent qui empêche la fragmentation du fil de cuivre. Si un tel stérilet résoud le problème de la fragmentation du fil de cuivre, il n'apporte qu'une solution imparfaite en ce qui concerne son aptitude à s'adapter à la forme de la cavité utérine, car si sa souplesse est améliorée par rapport aux stérilets précédemment proposés, cette souplesse n'est pas suffisante pour permettre son adaptation dans tous les cas aux dimensions variables des cavités utérines des patientes traitées. En outre, un tel stérilet ne résoud pas le problème posé par la diminution dans le temps de la désorption des ions cuivre. Les recherches menées dans le but d'apporter une solution à l'un et l'autre de ces deux problèmes ont abouti à la mise au point d'un dispositif intra-utérin constitué par une matrice résiliente et élastique obtenue par moulage par injection d'un copolymère résultant du greffage réticulant d'un polymère hydrophile sur un polymère de base hydrophobe, dans laquelle est occluse une charge de cuivre sous forme de poudre de cuivre très pure répartie de façon homogène dans ladite matrice. En raison de la structure réticulée et du caractère hydrophile conférés à la matrice, il semblerait que les ions cuivre désorbent du dispositif intra-utérin à une vitesse quasi-constante tant que du cuivre métal demeure disponible, ce qui devrait militer en faveur d'une augmentation de la longévité de tels stérilets par rapport à celle des stérilets proposés précédemment. Toutefois, malgré leur souplesse, ces dispositifs intra-utérins posent encore des problèmes d'adaptation aux dimensions des cavités utérines des patientes traitées puisque les résultats cliniques ont fait apparaître un taux d'expulsions de l'ordre de 3,5 % dues en grande partie à leur mauvaise adaptation aux dimensions de la cavité utérine, mais aussi, dans un certain nombre de cas, à la mauvaise biocompatibilité du polyéthylène, qui provoque des réactions d'intolérance qui sont à l'origine du rejet du stérilet.

La présente invention s'est en conséquence donné pour but de pourvoir à un dispositif intra-utérin qui répond mieux aux nécessités de la pratique que les dispositifs intra-utérins proposés dans l'Art antérieur, notamment en ce qu'il est souple, élastique et déformable, en ce qu'il est, au moins en partie, réalisé en matériau biocompatible, en ce qu'il est adaptable à la demande, aux dimensions de la cavité utérine de chaque patiente, tout en étant constitué d'éléments pouvant être produits aisément à l'échelle industrielle, en ce qu'il présente une action contraceptive accrue par rapport à celle des stérilets proposés dans l'Art antérieur, et en ce que son action peut être contrôlée

- 3 -

pour être variable selon la localisation des diverses zones du dispositif intra-utérin.

La présente invention a pour objet un dispositif intra-utérin du type comportant une tige se terminant, à l'une de ses extrémités, par deux branches dont les extrémités portent chacune une protubérance ou analogue, tandis que l'autre extrémité de la tige est pourvue d'un fil d'éjection, la tige et les branches étant réalisées en matière plastique souple, par moulage par injection en une seule pièce et la tige portant une pluralité de perles, lequel dispositif intra-utérin est caractérisé en ce que la tige et les branches susdites constituent une armature souple qui est revêtue d'une mince couche de carbone, en ce que les protubérances portées par les branches de ladite armature sont réalisées en totalité ou en partie en carbone, en ce que la pluralité de perles est enfilée sur la tige, lesquelles perles sont constituées par, ou contiennent, une matière propre à se libérer progressivement dans la cavité utérine, dans les conditions qui règnent dans cette dernière, pendant une durée prolongée pouvant atteindre 5 ans et plus.

Selon un mode de réalisation avantageux du dispositif intra-utérin conforme à l'Invention, les perles enfilées sur la tige sont séparées les unes des autres par des rondelles en matière plastique souple.

Selon un autre mode de réalisation avantageux du dispositif intra-utérin conforme à l'Invention, les protubérances portées par les extrémités des branches de l'armature sont réalisées par moulage par injection en même temps que l'armature, dont elles font partie intégrante.

Selon un autre mode de réalisation avantageux du dispositif intra-utérin conforme à l'Invention, lesdites protubérances sont produites séparément et sont rapportées par collage sur les extrémités des branches de l'armature, qui sont pourvues à cet effet de micro-aspérités.

Conformément à la présente Invention, lesdites protubérances sont en matière plastique et comportent un revêtement en carbone, ou bien elles sont en carbone résultant de la carbonisation sous atmosphère neutre de structures en matière plastique moulée présentant la forme finale des protubérances, avantageusement revêtues d'un dépôt de pyrocarbone, ou en carbone vitreux.

Selon encore un autre mode de réalisation avantageux du dispositif intra-utérin conforme à l'Invention, les perles sont constituées soit par du métal pur, tel que cuivre notamment, apte à se libérer progressivement dans les conditions qui règnent dans la cavité utérine, éventuellement imprégné d'un composé organique également apte à se libérer progressivement dans la cavité utérine, soit par une matière poreuse telle que céramique, carbone poreux, métal poreux ou analogue, dans les pores de laquelle sont adsorbées ou incluses une ou plusieurs matières minérales et/ou organiques propres à se libérer progressivement dans la cavité utérine.

Selon encore un autre mode de réalisation avantageux du dispositif intra-utérin conforme à l'Invention, les perles sont conformées de manière à présenter une très grande surface extérieure d'accès facile et présenter la forme de rondelles, de profilés à ailettes ou autres.

On entend par "perles" au sens de la présente Invention, des structures de forme sensiblement circulaire, telles que des rondelles, de forme sensiblement sphérique ou de forme sensiblement cylindrique présentant ou non une structure poreuse et/ou présentant ou non une très grande surface extérieure qui leur est conférée par un profil approprié.

On entend par "protubérances" des structures sphériques, semi-sphériques, conoïdales, pyramidales, en forme d'arc de cercle, ou autres.

Selon une modalité avantageuse de l'Invention, appliquée au cas où les perles sont en carbone poreux, celles-ci sont constituées par du carbone obtenu par carbonisation de perles de matière plastique à pores ouverts présentant les dimensions recherchées.

Selon une autre modalité avantageuse de l'Invention, appliquée au cas où les perles sont en carbone poreux, celles-ci sont constituées par une matrice de carbone dans laquelle sont enrobées des fibres de carbone.

Selon encore une autre modalité avantageuse de l'Invention, appliquée au cas où les perles sont en métal pur, celles-ci résultent du frittage de grains dudit métal pour obtenir des perles de métal pur présentant des pores.

Selon un mode de réalisation avantageux du dispositif intra-utérin conforme à l'Invention, celui-ci est apte à présenter un effet de pile et à générer un micro-courant inoffensif pour l'organisme et propre à augmenter l'efficacité du dispositif intra-utérin, lorsque celui-ci est en place dans l'utérus, au contact du liquide salin présent dans ce dernier, lequel liquide joue le rôle d'électrolyte, cette aptitude résultant de la présence, dans le dispositif, de produits métalliques ou minéraux conducteurs de l'électricité.

Selon encore un autre mode de réalisation avantageux du dispositif intra-utérin conforme à l'Invention, la tige comporte à son extrêmité libre une butée propre à retenir les perles qui sont enfilées sur cette dernière.

Selon une modalité avantageuse de ce mode de réalisation, la butée est formée par écrasement à chaud de l'extrêmité libre de la tige, pour donner à ladite extrêmité une largeur supérieure au diamètre de la tige, qui bloque les perles sur cette dernière.

Selon un mode de réalisation particulièrement préféré de l'Invention, les perles enfilées sur la tige sont constituées et/ou imprégnées par des matériaux différents selon leur localisation sur la tige.

Pour faciliter la mise en place du dispositif intra-utérin selon l'invention dans l'utérus et éviter au cours de celle-ci le risque accidentel d'un léger enfoncement des protubérances portées par les extrémités des branches dudit dispositif, dans le fond de l'utérus, qui pourrait gêner le déploiement des branches, il est avantageux, selon un mode de réalisation particulier de l'invention, de pourvoir le dispositif intra-utérin conforme à l'invention d'un moyen de butée propre à éviter l'enfoncement des protubérances dans le fond de l'utérus et à permettre le déploiement aisé des branches qui portent lesdites protubérances, à l'intérieur de l'utérus.

Conformément à ce mode de réalisation, le dispositif intra-utérin comprend un bras réalisé en le même matériau souple que l'armature du dispositif intra-utérin formée par la tige et les deux branches portées par l'une des extrémités de cette dernière, lequel bras est ancré aux branches de manière à constituer un pont entre elles.

Selon une disposition avantageuse de ce mode de réalisation, le bras est ancré aux faces internes tournées l'une vers l'autre, des branches du dispositif intra-utérin.

Selon une modalité avantageuse de réalisation de cette disposition, l'ancrage du bras de butée sur les branches du dispositif intra-utérin est réalisé dans la zone terminale des branches, au voisinage des protubérances portées par l'extrémité de ces dernières, de préférence dans une zone comprise entre le 1/4 et le 1/6 de la longueur d'une branche, calculé à partir du point d'ancrage d'une protubérance sur l'extrémité de la branche correspondante.

Selon une autre disposition avantageuse de ce mode de réalisation, le bras de butée est réalisé simultanément avec l'armature du dispositif intra-utérin dont il fait partie intégrante, par moulage, de préférence par moulage par injection.

Selon encore une autre disposition avantageuse de ce mode de réalisation, le bras de butée présente une forme tubulaire de diamètre uniforme.

Selon une autre disposition avantageuse de ce mode de réalisation, le bras de butée présente dans sa zone médiane, qui correspond sensiblement à l'axe longitudinal du dispositif intra-utérin, une réduction de diamètre qui facilite la flexion du bras lorsque le dispositif intra-utérin se trouve à l'état replié, avant son déploiement dans l'utérus.

Selon encore une autre disposition avantageuse de ce mode de réalisation, le bras de butée comporte, de même que la tige et les branches qui forment l'armature du dispositif intra-utérin, un revêtement de carbone.

La présente invention a également pour objet un procédé de fabrication du dispositif intra-utérin défini dans ce qui précède, lequel procédé consiste à mouler par injection, dans un moule présentant une empreinte appropriée, une armature en matière plastique souple, déformable, comprenant une tige dont l'une des extrémités porte deux branches, puis à revêtir ladite armature d'une mince couche de carbone déposée par dépôt en phase vapeur, à produire des perles en matériau approprié par une technique convenable, percées d'un trou traversant de diamètre sensiblement correspondant à celui de la tige de l'armature, à enfiler lesdites perles sur la tige, en un nombre correspondant aux dimensions de la cavité utérine à appareiller, puis à écraser à chaud l'extrémité libre de la tige pour en augmenter la largeur et lui faire jouer, sous cette forme, le rôle de butée de verrouillage des perles enfilées sur la tige, puis à percer un trou dans cette butée et à y passer des fils d'éjection.

Selon un mode de mise en oeuvre avantageux du procédé conforme à l'Invention, on pourvoit chacune des extrémités libres des branches de l'armature, d'une protubérance de forme appropriée.

Selon une modalité du procédé conforme à l'invention, lesdites protubérances sont venues de moulage avec l'armature puis revêtues, en même temps que celle-ci, d'une mince couche de carbone .

Selon une autre modalité du procédé conforme à l'invention, les protubérances sont réalisées séparément en matériau approprié, puis collées aux extrémités des branches, qui sont pourvues de microaspérités venues de moulage.

Conformément à une disposition avantageuse de ce procédé, les protubérances réalisées séparément sont obtenues à partir de structures en matière plastique de forme appropriée, réalisées par moulage, qui sont carbonisées en atmosphère neutre, pour produire des structures en carbone, qui sont ensuite revêtues d'un dépôt de pyrocarbone parfaitement étanche et lisse, totalement inerte chimiquement, et biologiquement, par craquage de méthane à 2000° sous pression réduite.

Conformément à une autre disposition avantageuse de ce procédé, les protubérances sont réalisées en carbone vitreux, puis fixées par collage aux extrémités des branches.

Selon un mode de mise en oeuvre avantageux du procédé conforme à l'invention, l'armature que l'on moule par injection dans un moule présentant une empreinte appropriée, est une armature en matière plastique souple, déformable, comprenant une tige dont l'une des extrémités porte deux branches, elles-mêmes reliées entre elles par un bras qui forme un pont entre elles.

Selon un autre mode de mise en oeuvre avantageux du procédé, les perles sont réalisées en métal pur, et notamment en un métal propre à libérer ses ions métalliques dans les conditions qui règnent dans la cavité utérine, pour y exercer une action contraceptive.

Selon encore un autre mode de mise en oeuvre avantageux du procédé, les perles sont réalisées en métal pur, par frittage de grains dudit métal dans le but de ménager des pores dans la structure, qui est ensuite moulée en forme de sphères poreuses.

Selon un autre mode de mise en oeuvre avantageux du procédé conforme à l'invention, les perles sont réalisées, par moulage, en un matériau poreux tel que céramique.

Selon encore un autre mode de mise en oeuvre avantageux du procédé conforme à l'invention, les perles sont réalisées à partir de boules en matière plastique qui sont carbonisées en atmosphère neutre, pour former des perles en carbone

0147274

- 9 -

poreux.

Conformément à un autre mode de mise en oeuvre avantageux du procédé conforme à la présente invention, les perles poreuses sont réalisées par moulage d'un matériau composite constitué par une matrice de carbone dans laquelle sont noyées des fibres de carbone.

Selon une modalité avantageuse du procédé conforme à l'invention, les perles en matière poreuse sont imprégnées d'une ou de plusieurs substances à action thérapeutique et/ou à action contraceptive.

Conformément à une disposition avantageuse du procédé conforme à l'Invention, l'imprégnation des perles en matière poreuse par au moins une substance appropriée est réalisée de manière à remplir totalement les pores desdites perles.

Conformément à une autre disposition avantageuse du procédé conforme à l'Invention, l'imprégnation des perles en matière poreuse par au moins une substance appropriée est réalisée de manière à ne remplir que partiellement les pores desdites perles afin d'améliorer la surface spécifique d'échange avec le milieu liquide ambiant de la cavité utérine, auquel cas la ou les substances d'imprégnation sont évaporées sous vide pendant la phase d'imprégnation.

Conformément à l'invention, les perles en matière poreuse sont imprégnées d'un métal à action contraceptive, tel que du cuivre notamment.

Egalement conformément à l'invention, les perles en matière poreuse sont imprégnées d'une substance présentant une activité thérapeutique telle qu'une hormone, en particulier de la progestérone, ou un antibiotique ou encore un agent anti-contractile.

Egalement conformément à l'invention, les perles en matière poreuse sont imprégnées à la fois de métal et d'une substance présentant une activité thérapeutique.

Egalement conformément au procédé qui fait l'objet de la présente Invention, les perles, en matière compacte ou poreuse, sont moulées pour leur donner une forme propre à augmenter leur surface extérieure, telle qu'empilement de rondelles ou profilé à ailettes, notamment, un tel profilé pouvant être obtenu soit par moulage, soit par usinage.

Conformément à un mode de mise en oeuvre avantageux du procédé conforme à la présente invention, on enfile sur la tige de l'armature une pluralité de perles et entre deux perles consécutives on enfile des rondelles de séparation en matière plastique souple qui ont pour rôle de séparer lesdites perles les unes des autres afin que, lors de la flexion de l'armature, au moment de sa mise en place dans la cavité utérine, les perles ne puissent pas se détériorer mutuellement par contact.

Conformément à un autre mode de mise en oeuvre avantageux du procédé conforme à l'Invention, on enfile sur la tige de l'armature, soit des perles présentant toutes la même constitution, soit par paires successives, alternativement, une perle constituée par ou contenant un métal à action contraceptive et une perle contenant une substance présentant une activité thérapeutique ; soit en localisant les perles contenant une substance présentant une activité thérapeutique dans une zone de la tige et les perles comprenant une substance à action contraceptive dans une autre zone de la tige.

Egalement conformément au procédé objet de la présente invention, les perles comprenant une substance à action contraceptive enfilées sur une même tige ou sur une zone de cette dernière, peuvent être alternativement des perles réalisées en matière à action contraceptive telle qu'un métal pur approprié, notamment le cuivre, et des perles en matière poreuse imprégnées de matière à action contraceptive, dans le but d'assurer un dosage approprié aux besoins spécifiques de chaque patiente, du taux de libération de la matière à action contraceptive dans la cavité utérine.

Outre les dispositions qui précèdent, l'Invention comprend encore d'autres dispositions, qui ressortiront de la description qui va suivre.

L'invention sera mieux comprise à l'aide du complément de description qui va suivre, qui se réfère aux dessins annexés dans lesquels :

la Figure 1a représente en vue en coupe axiale, un mode de réalisation du dispositif intra-utérin conforme à l'Invention,

la Figure 1b représente, également en vue en coupe axiale, un autre mode de réalisation du dispositif intra-utérin conforme à l'Invention,

la Figure 2a représente une vue en coupe axiale d'un mode de réalisation d'une perle conforme à la présente Invention, dont

la Figure 2b est une vue en coupe transversale,

la Figure 3a représente une vue latérale d'un autre mode de réalisation d'une perle conforme à la présente Invention, dont

la Figure 3b est une vue en coupe transversale,

la Figure 4 est une vue en coupe axiale d'un dispositif intra-utérin inclus dans son dispositif de mise en place,

les Figures 5 et 7 représentent des vues en coupe axiale de deux modes de réalisation de dispositifs intra-utérins pourvus d'un bras de butée et

la Figure 6 est une vue en coupe axiale d'un dispositif intra-utérin pourvu d'un bras de butée, à l'état replié, préalablement à son éjection hors du dispositif de protection et de mise en place dans lequel il est contenu, et à sa mise en place dans l'utérus.

Il doit être bien entendu, toutefois, que ces dessins et les parties descriptives correspondantes, sont donnés uniquement à titre d'illustration de l'objet de l'invention, dont ils ne constituent en aucune manière une limitation.

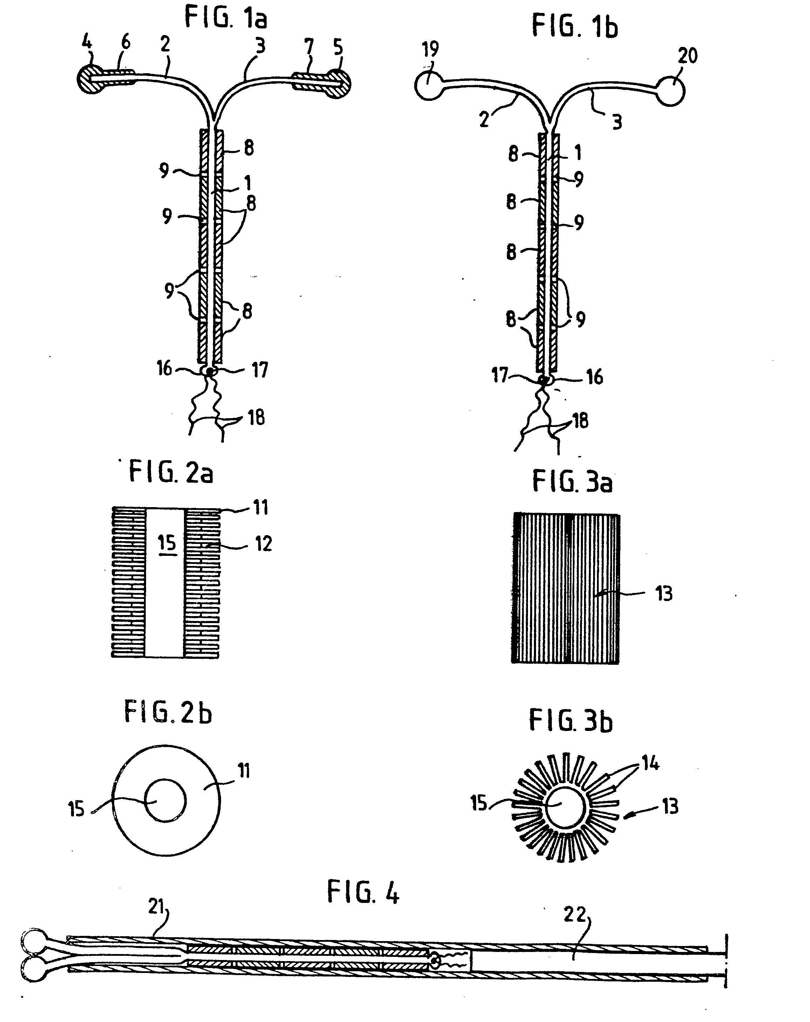

Le dispositif intra-utérin représenté à la Figure 1 prêt à l'emploi, comprend une armature en matière plastique souple, flexible et déformable, obtenue par moulage, notamment par injection, la matière plastique pouvant être, par exemple mais non limitativement, du polyéthylène ou du PTFE. Cette armature se compose d'une tige 1 qui porte à l'une de ses extrêmités, deux branches 2 et 3. Le diamètre de l'armature 1-2-3 peut être, par exemple de 1 mm, mais peut être inférieur ou supérieur si on le désire. La forme des branches 2, 3 est telle qu'elles puissent être aisément repliées sensiblement dans le prolongement de la tige 1 lorsque le dispositif intra-utérin est inclus dans son dispositif de mise en place (Cf. Figure 4). Chacune des deux branches 2, 3 porte à son extrêmité libre, une protubérance 4, 5, de 2 à 4 mm de large, de préférence. Dans le mode de réalisation représenté à la Figure 1, les protubérances 4, 5 sont réalisées séparément et sont pourvues d'un manchon 6, 7 à l'aide duquel elles sont enfilées sur les extrêmités des branches 2, 3, auxquelles elles sont fixées par la coopération d'une couche d'adhésif appliquée intérieurement sur la face intérieure des manchons 6, 7 et de micro-aspérités portées par lesdites extrêmités des branches 2, 3. Les protubérances 4, 5 rapportées sur les branches 2, 3 sont en carbone, par exemple en carbone vitreux ou en carbone obtenu par carbonisation en atmosphère neutre, de structures de forme appropriée moulées en matière plastique telle que Téflon, polycarbonate, résine phénolique, résine furanique, etc..., lesdites protubérances en carbone obtenues par carbonisation étant recouvertes d'un dépôt de pyrocarbone parfaitement étanche et lisse obtenu, par exemple, par craquage de méthane à 2000°C sous pression réduite, un tel revêtement étant totalement inerte chimiquement et biologiquement.

L'armature 1, 2, 3 est revêtue d'une mince couche de carbone dont l'épaisseur est de l'ordre de 0,1 micron par exemple, qui est déposé par le procédé connu sous le nom de PVD (physical vapor deposition), c'est à dire dépôt physique en phase vapeur, un tel dépôt étant réalisé soit par sublimation de carbone sous vide, soit par projection de carbone arraché sur la cible de base à l'aide d'un faisceau d'électrons, soit par tout autre procédé approprié.

Sur la tige centrale est enfilée une pluralité de perles 8 percées d'un trou 15, qui sont en matière rigide et qui sont séparées les unes des autres par des rondelles 9 en matière plastique souple, telle que polyéthylène souple, par exemple, enfilées en même temps que les perles 8, qui ont pour rôle d'empêcher que les perles 8 ne se détériorent mutuellement par contact lors de mouvements angulaires réciproques intervenant lors de la flexion du dispositif intra-utérin, au moment de sa mise en place. L'ensemble constitué par les perles 8 et les rondelles 9 interposées, conserve, malgré la rigidité des perles qui le composent, une souplesse en flexion, notamment en raison de la discontinuité des perles 8 sur la tige 1.

Les perles 8 ont, de préférence, une forme généralement cylindrique. Il peut, cependant, être avantageux de leur conférer une très grande surface extérieure, directement accessible, en les réalisant, par exemple, sous la forme d'un empilement de rondelles ou d'un profilé à ailettes. Les Figures 2a et 2b montrent respectivement en coupe axiale et en coupe transversale, un empilement de rondelles 11-12 de diamètres différents alternés : à titre d'exemple non limitatif, les rondelles 11 peuvent présenter un diamètre de 2,5 mm et les rondelles 12 peuvent présenter un diamètre de 1,5 mm; l'épaisseur de ces rondelles peut être plus ou moins grande, et être aussi faible, notamment que 0,01 à 0,02 mm.

Les Figures 3a et 3b montrent, respectivement en vue latérale et en coupe transversale, un profilé 13 à ailettes

0147274

- 14 -

dont les ailettes 14 venues de moulage ou obtenues par usinage (par exemple par étirage) confèrent à la perle 13 une très grande surface extérieure, aisément accessible.

Les perles 8, 11-12 ou 13-14 mises en place sur la tige 1 avec les rondelles séparatrices 9 interposées, sont bloquées en position sur ladite tige 1 en écrasant à chaud l'extrémité libre de la tige 1, pour lui conférer une largeur supérieure au diamètre de la tige 1 et au diamètre des trous 15 ménagés dans les perles pour permettre de les enfiler sur la tige 1, et pour assurer leur verrouillage sur celle-ci.

Cette extrémité écrasée 16 est percée d'un trou 17 dans lequel sont passés des fils d'éjection 18 propres à permettre l'extraction du dispositif intra-utérin en cas de besoin ou à la fin de sa période d'activité.

Les perles enfilées sur la tige 1 ont pour rôle de libérer une substance à action contraceptive ou thérapeutique. Elles peuvent présenter une composition et une structure adaptées aux besoins spécifiques auxquels doit répondre le dispositif intra-utérin conforme à la présente invention.

En particulier, lesdites perles peuvent être constituées par du métal pur tel que cuivre par exemple, et être obtenues par moulage d'un métal compact. Elles peuvent aussi être constituées par du métal pur poreux résultant du moulage sous forme de perles, d'un métal obtenu par frittage de grains dudit métal, un tel processus de fabrication conférant aux perles obtenues une structure poreuse. Elles peuvent également être formées d'un support poreux en carbone poreux ou en céramique, par exemple, ou encore en matériau composite carbone-carbone (c'est à dire une matrice de carbone dans laquelle sont enrobées des fibres de carbone, les densités des fibres et de la matrice étant choisies en fonction des besoins spécifiques). Les perles en métal poreux présentent une grande surface spécifique qui améliore la constance de libération des ions métalliques dans le milieu ambiant de la cavité utérine. Toutefois, de telles perles en métal poreux peuvent être imprégnées d'une autre substance active, telle qu'une substance à activité thérapeutique (hormone, antibiotique, etc...) ou une substance à action anti-contractile (anti-prostaglandines par exemple).

Les perles formées d'un support creux en carbone poreux ou en matériau composite constitué par une matrice de carbone dans laquelle sont noyées des fibres de carbone ou en un autre matériau poreux tel que céramique, par exemple, sont imprégnées de constituant actif tel que du métal à action contraceptive, notamment de cuivre, ou d'une substance présentant une activité thérapeutique, ou à la fois d'un métal à action contraceptive et d'une substance présentant une activité thérapeutique (hormone telle que progestérone, par exemple, antibiotique tel que "Gentalline" ou "Flagyl" par exemple, substance à action anti-contractile, etc...). Les perles en carbone poreux sont avantageusement (mais non limitativement) obtenues par carbonisation d'une matière plastique comportant des pores ouverts présentant les dimensions de pores souhaitées et en nombre voulu. Le choix des dimensions des pores des perles poreuses présente une grande importance, car ces dimensions conditionnent l'activité du dispositif intra-utérin, et notamment le dosage du taux de libération et la vitesse de libération du constituant actif dans le milieu ambiant de la cavité utérine. La proportion de constituant, tel que cuivre par exemple, contenue dans les pores du support poreux, peut être comprise entre 0 et 90 % en poids . Les pores peuvent être complètement remplis de constituant actif ou présenter des lacunes pour augmenter la surface spécifique de contact avec le milieu liquide ambiant de la cavité utérine. Dans ce dernier cas, les lacunes sont obtenues, par exemple, par évaporation sous vide de la substance introduite dans les pores du support poreux, lors de l'opération d'imprégnation.

Dans le mode de réalisation représenté à la Figure 1b, les protubérances portées par les extrémités des branches 2, 3 sont des protubérances 19, 20 en matière plastique, venues de moulage avec la tige 1 et les branches 2, 3. Ces protubérances sont revêtues, en même temps que la tige 1 et les branches 2, 3, d'une mince couche de carbone par le procédé PVD mentionné plus haut.

A noter que les branches 2, 3 avec leurs protubérances 4-5 ou 19-20 terminales servent, ainsi qu'il est connu, à l'ancrage du dispositif intra-utérin dans l'utérus, à la différence près que l'ancrage réalisé est plus efficace et plus fiable que les barres d'ancrage proposées dans l'Art antérieur, en raison de la présence des protubérances et aussi en raison de la présence du revêtement en carbone qui est parfaitement biocompatible et évite les réactions inflammatoires ou infectieuses qui peuvent aboutir au rejet du dispositif intra-utérin, que l'on observe avec les stérilets connus dans l'Art antérieur.

La réalisation des perles en carbone poreux imprégnées de métal à action contraceptive, et notamment de cuivre, procure un effet supplémentaire, représenté par le fait que, le carbone et le métal étant conducteurs de l'électricité, lorsque le dispositif conforme à l'invention est en place dans la cavité utérine, en présence du milieu liquide salin ambiant, il se produit un effet de pile qui engendre un micro-courant électrique de faible tension, inoffensif pour l'organisme, qui potentialise l'action anti-nidatoire du dispositif conforme à la présente invention, la tension pouvant être réglée à volonté en choisissant judicieusement les matériaux conducteurs. Par exemple, dans le cas où les perles sont en carbone poreux et sont imprégnées de cuivre, les tensions respectives de ces matériaux sont de - 0,2 V et - 0,33 V, ce qui constitue une pile très faible et donc sans danger pour l'organisme, tout en étant suffisante pour potentialiser l'action anti-nidatoire du dispositif conforme à l'Invention.

Le nombre de perles enfilées sur la tige 1 est fonction des dimensions à conférer au dispositif intra-utérin conforme à l'invention, elles-mêmes fonction des dimensions de la cavité utérine de chaque patiente. Les perles enfilées sur la tige 1 peuvent être identiques entre elles ou différentes. Dans ce dernier cas, les perles de composition et/ou de structure différentes peuvent être enfilées dans un ordre variable en fonction des résultats recherchés. C'est ainsi que l'on peut faire alterner des perles à action contraceptive et des perles à action thérapeutique. C'est ainsi, également, que l'on peut localiser les perles présentant une action thérapeutique sur une partie de la tige, par exemple sur la partie proche des branches, et localiser les perles présentant une action contraceptive sur une autre partie de la tige, permettant, de ce fait l'adaptation du dispositif intra-utérin conforme à l'invention à diverses fonctions chimico-biologiques et la mise à la disposition du corps médical et des patientes concernées, d'un dispositif à large spectre d'utilisation, capable de s'adapter à tous les types de réactions pathologiques ou mécaniques susceptibles d'être rencontrées chez les sujets porteurs.

Le dispositif intra-utérin est mis en place, d'une façon connue, à l'aide du dispositif de mise en place dans lequel il est inclus lors de sa commercialisation, lequel comprend un tube-poussoir 21 et une tige d'éjection 22.

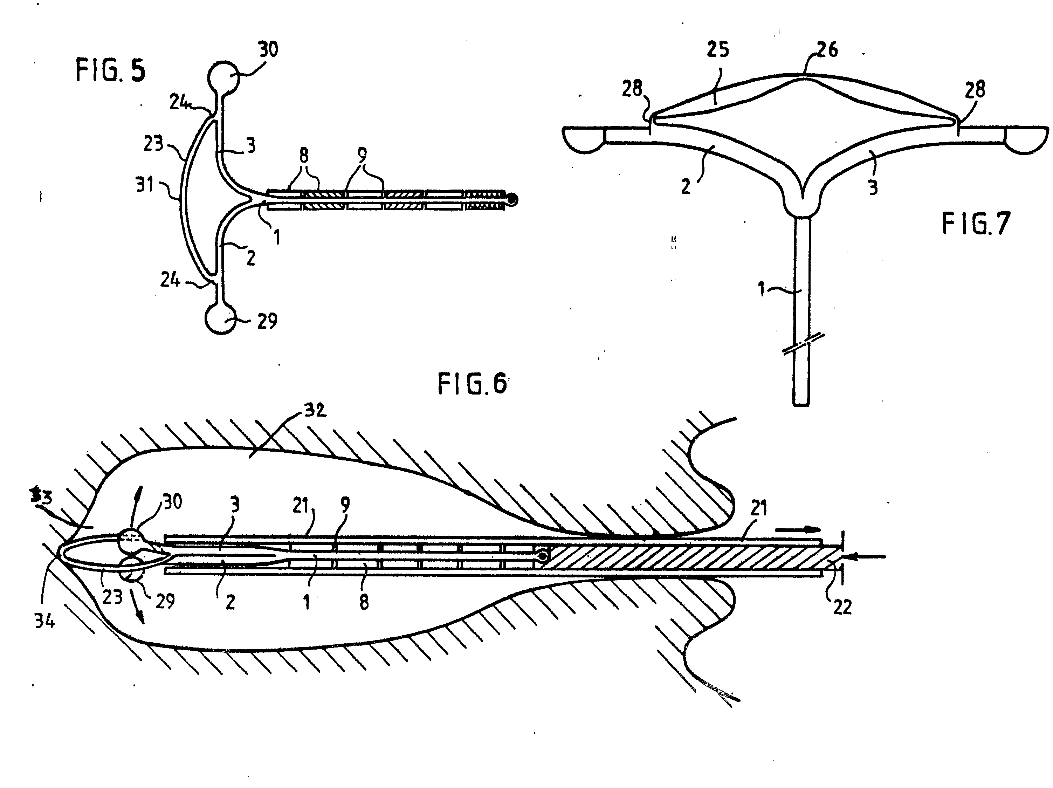

Le dispositif intra-utérin représenté aux figures 5 à 7 se distingue du dispositif intra-utérin représenté aux figures 1 à 4 par le fait que les deux branches 2 et 3 sont reliées l'une à l'autre par un bras qui forme un pont entre elles.

Le bras 23 qui relie les branches 2, 3 conformément à la figure 5, présente un diamètre sensiblement égal sur toute sa longueur, tandis que le bras 25 qui relie les branches 2, 3 conformément à la figure 7, présente un diamètre variable en ce que sa zone médiane 26 est étranglée par rapport au diamètre du reste dudit bras 25, une telle réduction de diamètre dans la zone médiane 26 du bras facilitant sa flexion à l'emplacement de diamètre réduit, notamment lorsque le dispositif intra-utérin se trouve à l'état replié dans son tube protecteur 21 qui sert en même temps à la mise en place du dispositif intra-utérin dans l'utérus.

De même, pour permettre une meilleure articulation du bras 25 par rapport aux branches 2, 3 du dispositif intra-utérin, il peut être avantageux que les extrêmités 28, 28 du bras 25, par l'intermédiaire desquelles celui-ci vient s'ancrer respectivement sur les branches 2, 3, présentent également un diamètre réduit (Cf. figure 7).

Quel que soit le mode de réalisation adopté pour le bras 23 ou 25 (Cf. respectivement figures 5 et 7), l'ancrage des extrémités, respectivement 24, 24 ou 28, 28 dudit bras sur les faces internes tournées l'une vers l'autre des branches 2, 3, est venu de moulage, le bras étant réalisé en même temps que l'armature 1-2-3, par moulage, de préférence par injection.

L'emplacement des branches 2, 3, choisi pour l'ancrage du bras 23 ou 25, est aussi proche que possible des protubérances 29, 30 portées par l'extrêmité extérieure de chacune des branches 2, 3, pour les raisons qui seront exposées plus loin, et la longueur du bras 23 ou 25 est telle qu'il forme de préférence un arc de cercle convexe saillant vers l'extérieur, lorsque le dispositif intra-utérin est en place dans l'utérus,avec les branches 2, 3 totalement déployées.

Du fait de son ancrage sur les branches 2, 3 aussi près que possible des protubérances 29-30 et de sa réalisation en forme d'arc de cercle saillant vers l'extérieur, c'est-à-dire de longueur sensiblement supérieure à celle de sa corde, le bras 23 ou 25 avance, lorsque le dispositif intra-utérin conforme à

0147274

- 19 -

l'invention est à l'état replié, et que le bras 23 ou 25 est, par conséquent, lui-même replié, au-delà des protubérances 29-30, en sorte que c'est la zone 34 saillante du bras, qui s'étend de part et d'autre de sa zone médiane 31 ou 26, respectivement, qui entre en contact avec le fond 33 de l'utérus 32, servant ainsi de butée propre à empêcher que les protubérances 29, 30 n'entrent en contact avec ledit fond 33 de l'utérus. De ce fait, lorsque le dispositif intra-utérin est progressivement libéré du tube 21 par poussée à l'aide de la tige-poussoir 22 d'éjection, les branches 2, 3 peuvent se déployer librement puisque le rôle de butée du bras 23 ou 25 évite que les protubérances 29, 30 ne viennent se bloquer dans le fond de l'utérus, comme cela pourrait être le cas en l'absence du bras 23 ou 25. L'opération de mise en place du dispositif intra-utérin est ainsi considérablement simplifiée, aussi bien pour le médecin que pour la patiente.

L'on obtient, conformément à la présente invention, un dispositif intra-utérin souple, déformable, dont la vitesse de désorption du métal contraceptif est rigoureusement contrôlée et dont l'adaptabilité aux diverses réactions mécaniques, biologiques ou pathologiques des patientes, est très grande, et pratiquement illimitée. En outre, le dispositif intra-utérin est biocompatible en raison de la présence sur l'armature d'un revêtement de carbone et du fait que les perles peuvent également être réalisées de telle sorte qu'elles comportent un support poreux biocompatible, notamment en carbone. De plus, la surface spécifique activée du dispositif intra-utérin conforme à l'Invention est considérablement augmentée par rapport aux dispositifs visant au même but antérieurement connus, sa longévité est sensiblement plus grande que celle des dispositifs de l'Art antérieur et sa biocompatibilité est encore augmentée, dans le cas où l'on utilise des perles-supports en carbone poreux, en raison du fait que le carbone poreux est colonisable. En outre, le dispositif résoud pratiquement les problèmes inflammatoires et infectieux d'une part en raison de sa biocompatibilité et d'autre part par la possibilité qu'il donne de réaliser des sortes de mini-implants d'antibiotiques, de progestatifs ou de substances anti-contractiles. Enfin, ce dispositif présente une grande facilité de mise en place, sécurisante aussi bien pour le médecin que pour la patiente.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

0147274

- 21 -

<u>REVENDICATIONS</u>

1°) Dispositif intra-utérin du type comportant une tige se terminant à l'une de ses extrémités par deux branches dont les extrémités portent chacune une protubérance ou analogue, tandis que l'autre extrémité de la tige est pourvue d'un fil d'éjection, dispositif dans lequel la tige et les branches sont réalisées en matière plastique souple, par moulage par injection en une seule pièce, et la tige porte une pluralité de perles, lequel dispositif intra-utérin est caractérisé en ce que la tige (1) et les branches (2, 3) susdites constituent une armature souple qui est revêtue d'une mince couche de carbone, en ce que les protubérances (4, 5 ou 19, 20) portées par les branches (2,3) de ladite armature sont réalisées en totalité ou en partie en carbone, en ce que la pluralité de perles (8) est enfilée sur la tige (1), lesquelles perles (8) sont constituées par ou contiennent une matière propre à se libérer progressivement dans la cavité utérine, dans les conditions qui règnent dans cette dernière, pendant une durée prolongée pouvant atteindre 5 ans et plus.

2°) Dispositif selon la Revendication 1, caractérisé en ce que les perles (8) enfilées sur la tige sont séparées les unes des autres par des rondelles (9) en matière plastique souple.

3°) Dispositif selon l'une quelconque des Revendications 1 et 2, caractérisé en ce que les protubérances (19, 20) portées par les extrêmités des branches de l'armature sont réalisées par moulage par injection en même temps que l'armature, dont elles font partie intégrante.

4°) Dispositif selon l'une quelconque des Revendications 1 et 2, caractérisé en ce que lesdites protubérances (4, 5) sont produites séparément et sont rapportées par collage sur les extrêmités des branches (2, 3) de l'armature,qui sont pourvues à cet effet de micro-aspérités.

5°) Dispositif selon l'une quelconque des Revendi-

0147274

- 22 -

cations 1 à 3, caractérisé en ce que lesdites protubérances (19, 20) sont en matière plastique et comportent un revêtement en carbone.

6°) Dispositif selon l'une quelconque des Revendications 1, 2 et 4, caractérisé en ce que lesdites protubérances sphériques (4, 5) sont en carbone résultant de la carbonisation sous atmosphère neutre de structures en matière plastique moulée présentant la forme finale des protubérances, avantageusement revêtues d'un dépôt de pyrocarbone.

7°) Dispositif selon l'une quelconque des Revendications 1, 2 et 4, caractérisé en ce que lesdites protubérances sont en carbone vitreux.

8°) Dispositif selon l'une quelconque des Revendications 1 à 7, caractérisé en ce que les perles sont constituées par du métal pur, tel que le cuivre notamment, apte à se libérer progressivement dans la cavité utérine.

9°) Dispositif selon l'une quelconque des Revendications 1 à 7, caractérisé en ce que les perles sont constituées par du métal pur poreux imprégné d'au moins une substance thérapeutique, aptes, tous deux, à se libérer progressivement dans la cavité utérine.

10°) Dispositif selon l'une quelconque des Revendications 1 à 7, caractérisé en ce que les perles sont en une matière poreuse telle que céramique ou carbone poreux ou analogue, dans les pores de laquelle sont adsorbées ou incluses une ou plusieurs matières minérales et/ou organiques aptes à se libérer progressivement dans la cavité utérine.

11°) Dispositif selon l'une quelconque des Revendications 1 à 10, caractérisé en ce que les perles sont conformées de manière à présenter une très grande surface extérieure d'accès facile et présenter la forme de rondelles (11, 12), de profilés à ailettes (13, 14) ou autres.

12°) Dispositif selon l'une quelconque des Revendications 1 à 11, caractérisé en ce qu'il est apte à présenter un effet de pile et à générer un micro-courant inoffensif pour l'organisme et propre à augmenter l'efficacité du dispositif intra-utérin, lorsque celui-ci est en place dans
l'utérus, au contact du liquide salin présent dans ce
dernier, lequel liquide joue le rôle d'électrolyte, cette
aptitude résultant de la présence, dans le dispositif, de
produits métalliques ou minéraux conducteurs de l'électricité.

13°) Dispositif selon l'une quelconque des Revendications 1 à 12, caractérisé en ce que la tige (1) comporte
à son extrémité libre une butée (16) propre à retenir les
perles qui sont enfilées sur cette dernière.

14°) Dispositif selon la Revendication 13, caractérisé en ce que la butée (16) est formée par écrasement à
chaud de l'extrémité libre de la tige (1), pour lui conférer
une largeur supérieure au diamètre de la tige, qui bloque
les perles (8) sur cette dernière.

15°) Dispositif selon l'une quelconque des Revendications 1 à 14, caractérisé en ce que les perles (8) enfilées
sur la tige sont constituées et/ou imprégnées par des
matériaux différents selon leur localisation sur la tige.

16°) Dispositif intra-utérin selon l'une quelconque
des revendications 1 à 15 , c a r a c t é r i s é
en ce qu'il comprend un bras (23, 25) réalisé en le même
matériau souple que l'armature du dispositif intra-utérin
formée par la tige (1) et les deux branches (2, 3) portées
par l'une des extrémités de cette dernière, lequel bras
(23, 25) est ancré aux branches (2, 3) de manière à constituer un pont entre elles.

17°) Dispositif intra-utérin selon la revendication
16, caractérisé en ce que ledit bras (23 ou 25) est ancré
aux faces internes tournées l'une vers l'autre, des branches
(2, 3) du dispositif intra-utérin.

18°) Dispositif intra-utérin selon la revendication
17, caractérisé en ce que l'ancrage du bras de butée (23 ou
25) sur les branches (2, 3) du dispositif intra-utérin est réalisé dans la zone terminale des branches (2, 3) , au voisinage des protubérances (29, 30) portées par l'extrêmité de ces dernières.

19°) Dispositif intra-utérin selon l'une quelconque des revendications 16 à 18, caractérisé en ce que la longueur du bras de butée (23 ou 25) est telle que lorsque le dispositif intra-utérin est à l'état replié, le bras avance au-delà des protubérances (29, 30) portées par les branches (2, 3) dudit dispositif intra-utérin et qu'il forme un arc de cercle convexe saillant vers l'extérieur lorsque le dispositif intra-utérin est prêt à l'emploi,avec ses branches déployées.

20°) Dispositif intra-utérin selon l'une quelconque des revendications 16 à 19, caractérisé en ce que le bras de butée (23 ou 25) est réalisé simultanément avec l'armature du dispositif intra-utérin dont il fait partie intégrante, par moulage, de préférence par moulage par injection.

21°) Dispositif intra-utérin selon l'une quelconque des revendications 16 à 20, caractérisé en ce que le bras de butée (23) présente une forme tubulaire de diamètre uniforme.

22°) Dispositif intra-utérin selon l'une quelconque des revendications 16 à 20, caractérisé en ce que le bras de butée (25) présente, dans sa zone médiane (26) qui correspond sensiblement à l'axe longitudinal du dispositif intra-utérin, une réduction de diamètre qui facilite la flexion du bras lorsque le dispositif intra-utérin se trouve à l'état replié, avant son déploiement dans l'utérus.

23°) Dispositif intra-utérin selon l'une quelconque des revendications 16 à 22, caractérisé en ce que les extrêmités (28) du bras de butée (23 ou 25), par l'intermédiaire desquelles celui-ci vient s'ancrer sur les branches (2, 3) du dispositif intra-utérin, présentent, par rapport au reste du bras, un diamètre réduit qui facilite la flexion du bras par rapport aux branches, lorsque le dispositif intra-utérin se trouve à l'état replié.

24°) Dispositif intra-utérin selon l'une quelconque des revendications 16 à 23, caractérisé en ce que le bras de butée (23 ou 25) comporte, de même que la tige (1) et les branches (2, 3) qui forment l'armature du dispositif intra-utérin, un revêtement de carbone.

25°) Procédé de fabrication du dispositif intra-utérin selon l'une quelconque des Revendications 1 à 24, caractérisé en ce qu'il consiste à mouler par injection dans un moule présentant une empreinte appropriée, une armature en matière plastique souple déformable comprenant une tige dont l'une des extrêmités porte deux branches, puis à revêtir ladite armature d'une mince couche de carbone déposée par dépôt en phase vapeur, à produire des perles en matériau approprié par une technique convenable, percées d'un trou traversant de diamètre sensiblement correspondant à celui de la tige de l'armature, à enfiler lesdites perles sur la tige, en un nombre correspondant aux dimensions de la cavité utérine à appareiller, puis à écraser à chaud l'extrêmité libre de la tige pour en augmenter la largeur et lui faire jouer, sous cette forme, le rôle de butée de verrouillage des perles enfilées sur la tige, puis à percer un trou dans cette butée et à y passer des fils d'éjection.

26°) Procédé selon la Revendication 25, caractérisé en ce qu'on pourvoit chacune des extrêmités libres des branches de l'armature, d'une protubérance de forme appropriée.

27°) Procédé selon la Revendication 26, caractérisé en ce que lesdites protubérances sont venues de moulage avec l'armature puis revêtues, en même temps que celle-ci, d'une mince couche de carbone.

28°) Procédé selon la Revendication 26, caractérisé en ce que les protubérances sont réalisées séparément en matériau approprié, puis collées aux extrêmités des branches, qui sont pourvues de micro-aspérités venues de moulage.

29°) Procédé selon la Revendication 28, caractérisé en ce que les protubérances réalisées séparément sont obtenues à partir de structures en matière plastique obtenues par moulage, et ayant la forme finale des protubérances, qui sont carbonisées en atmosphère neutre, pour produire des structures en carbone, qui sont ensuite revêtues d'un dépôt de pyrocarbone parfaitement étanche et lisse, totalement inerte chimiquement et biologiquement, par craquage de méthane à 2000° sous pression réduite, notamment.

30°) Procédé selon la Revendication 28, caractérisé en ce que les protubérances sont réalisées en carbone vitreux, puis fixées par collage aux extrémités des branches.

31°) Procédé selon l'une quelconque des Revendications 25 à 30, caractérisé en ce que les perles sont réalisées en métal pur, et notamment en un métal propre à libérer ses ions métalliques dans les conditions qui règnent dans la cavité utérine, pour y exercer une action contraceptive.

32°) Procédé selon l'une quelconque des Revendications 25 à 30 , caractérisé en ce que les perles sont réalisées en métal pur, par frittage de grains dudit métal dans le but de ménager des pores dans la structure, qui est ensuite moulée en forme de sphères poreuses.

33°) Procédé selon l'une quelconque des Revendications 25 à 30, caractérisé en ce que les perles sont réalisées, par moulage, en un matériau poreux tel que céramique.

34°) Procédé selon l'une quelconque des Revendications 25 à 30, caractérisé en ce que les perles sont réalisées à partir de boules en matière plastique qui sont carbonisées en atmosphère neutre, pour former des perles en carbone poreux.

35°) Procédé selon l'une quelconque des Revendications 25 à 30, caractérisé en ce que les perles poreuses sont réalisées par moulage d'un matériau composite constitué par une matrice de carbone dans laquelle sont noyées des fibres de carbone.

36°) Procédé selon l'une quelconque des Revendications 25 à 30 et 32 à 35, caractérisé en ce que les perles en matière poreuse sont imprégnées d'une ou de plusieurs substances à action thérapeutique et/ou à action contraceptive.

37°) Procédé selon la Revendication 36, caractérisé en ce que l'imprégnation des perles en matière poreuse par au moins une substance appropriée est réalisée de manière à remplir totalement les pores desdites perles.

38°) Procédé selon la Revendication 36, caractérisé en ce que l'imprégnation des perles en matière poreuse par au moins une substance appropriée est réalisée de manière à ne remplir que partiellement les pores desdites perles afin d'améliorer la surface spécifique d'échange avec le milieu liquide ambiant de la cavité utérine, auquel cas la ou les substances d'imprégnation sont évaporées sous vide pendant la phase d'imprégnation.

39°) Procédé selon l'une quelconque des Revendications 36 à 38, caractérisé en ce que les perles en matière poreuse sont imprégnées d'un métal à action contraceptive, tel que du cuivre notamment.

40°) Procédé selon l'une quelconque des Revendications 36 à 39, caractérisé en ce que les perles en matière poreuse sont imprégnées d'une substance présentant une activité thérapeutique, telle qu'une hormone, en particulier de la progestérone, ou un antibiotique ou encore une substance anti-contractile.

41°) Procédé selon l'une quelconque des Revendications 25 à 40, caractérisé en ce que les perles, en matière compacte ou poreuse, sont moulées pour leur donner une forme propre à augmenter leur surface extérieure, telle qu'empilement de rondelles ou profilé à ailettes, notamment, un tel profilé pouvant être obtenu soit par moulage, soit par usinage.

42°) Procédé selon l'une quelconque des Revendications 25 à 41, caractérisé en ce qu'on enfile sur la tige de l'armature une pluralité de perles et entre deux perles consécutives on enfile des rondelles de séparation en matière plastique souple qui ont pour rôle de séparer lesdites perles les unes des autres afin que, lors de la flexion de l'armature, au moment de sa mise en place dans la cavité utérine, les perles ne puissent pas se détériorer mutuellement par contact.

43°) Procédé selon l'une quelconque des Revendications 25 à 42, caractérisé en ce qu'on enfile sur la tige de l'armature soit des perles présentant toutes la même constitution ; soit par paires successives, alternativement une perle constituée par et/ou contenant un métal à action contraceptive et une perle contenant une substance présentant une activité thérapeutique ; soit en localisant les perles contenant une substance présentant une activité thérapeutique dans une zone de la tige et les perles comprenant une substance à action contraceptive, dans une autre zone de la tige ; soit alternativement des perles réalisées en matière à action contraceptive, telle qu'un métal pur approprié, notamment le cuivre, et des perles en matière poreuse imprégnées de matière à action contraceptive, dans le but d'assurer un dosage approprié aux besoins spécifiques de chaque patiente, du taux et de la vitesse de libération de la matière à action contraceptive dans la cavité utérine.

44°) Procédé selon la revendication 25 et l'une quelconque des revendications 26 à 43, caractérisé en ce que l'armature que l'on moule par injection dans un moule présentant une empreinte appropriée, est une armature en matière plastique souple, déformable, comprenant une tige dont l'une des extrémités porte deux branches, elles-mêmes reliées entre elles par un bras qui forme un pont entre elles.

FIG. 1a

FIG. 1b

FIG. 2a

FIG. 3a

FIG. 2b

FIG. 3b

FIG. 4

FIG. 5

30

24

23

3

31

8    9

1

2

24

29

FIG. 7

25    26

28    28

2    3

1

FIG. 6

32

33

30    3    21    9    21

23    29    2    1    8    22

34

2/2

0147274

Office européen
des brevets

**RAPPORT DE RECHERCHE EUROPEENNE**

**0147274**
Numéro de la demande

EP   84 40 2445

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| | | | A 61 F   5/47 |
| Y | DE-A-3 209 290  (BANSON ENTERPRISES)<br>* Page 11, ligne 4 - page 13, ligne 4; figures * | 1-44 | |
| | --- | | |
| Y | EP-A-0 035 593  (SIGRI)<br><br><br>* Page 3, ligne 20 - page 4, ligne 9 * | 1,3,5,<br>8,10,<br>13,16<br>27,29,<br>31,35,<br>44 | |
| | --- | | |
| Y | US-A-4 018 220  (L.C.R. EMMETT)<br>* Colonne 3, lignes 28-49; figure 7 * | 2,42 | |
| | | | **DOMAINES TECHNIQUES RECHERCHES (Int. Cl.4)** |
| A | | 10,16,<br>31,36<br>38,40,<br>42 | A 61 F<br>A 61 L<br>A 61 K |
| | --- | | |
| Y | DE-A-2 207 939  (SCHERING)<br><br>* Page 2, ligne 16 - page 3, ligne 23; page 4, lignes 18-25; figures * | 4,14,<br>28 | |
| | ---                    -/-. | | |

Le présent rapport de recherche a été établi pour toutes les revendications

| Lieu de la recherche<br>LA HAYE | Date d'achèvement de la recherche<br>18-03-1985 | Examinateur<br>WOLF C.H.S. |
|---|---|---|

Office européen
des brevets

**RAPPORT DE RECHERCHE EUROPEENNE**

EP 84 40 2445

| | **DOCUMENTS CONSIDERES COMME PERTINENTS** | | Page 2 |
|---|---|---|---|

| Catégorie | Citation du document avec indication, en cas de besoin, des parties pertinentes | Revendication concernée | CLASSEMENT DE LA DEMANDE (Int. Cl.4) |
|---|---|---|---|
| A | | 1,11, 12,15 26,31 32,43 | |
| | --- | | |
| Y | US-A-3 817 248 (R.G. BUCKLES et al.) | 9,10, 32,33 37,38 40 | |
| | * Colonne 5, ligne 54 - colonne 6, ligne 4 * | | |
| A | | 3-6,27 -29 | |
| | --- | | |
| Y | FR-A-2 202 437 (A.H. ROBINS COMPANY) * Page 3, lignes 2-18; figure 1 * | 11,41 | |
| | --- | | DOMAINES TECHNIQUES RECHERCHES (Int. Cl.4) |
| Y | US-A-3 820 535 (J.L. MARCO) | 12,15 43 | |
| | * Colonne 5, ligne 15 - colonne 6, ligne 14; figures * | | |
| A | | 1,4,8 11,26 28,31 41 | |
| | --- | | |
| Y | FR-A-2 165 714 (R. GAUD) * En entier * --- -/- | 16-24 44 | |

Le présent rapport de recherche a été établi pour toutes les revendications

| Lieu de la recherche LA HAYE | Date d'achèvement de la recherche 18-03-1985 | Examinateur WOLF C.H.S. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen
des brevets

**RAPPORT DE RECHERCHE EUROPEENNE**

**0147274**

Numéro de la demande

EP 84 40 2445

| | **DOCUMENTS CONSIDERES COMME PERTINENTS** | | Page 3 |

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-1 561 923 (P.E. LEBECH et al.)<br>* Page 2, colonne 2, ligne 40 - page 3, colonne 1, ligne 25; figures 1,2 *<br>--- | 22,23 | |
| Y | FR-A-2 253 537 (SCHERING)<br><br>* Page 9, ligne 18 - page 11, ligne 35; figures * | 36,39, 43 | |
| A | | 1,2,4, 8,12, 15,26, 28,31, 40,41 | |
| | --- | | |
| P,A | EP-A-0 117 163 (M. LEVRIER)<br><br><br><br>* En entier *<br>--- | 1,8,10 ,12,16 ,31,36 ,39,40 ,43,44 | DOMAINES TECHNIQUES RECHERCHES (Int. Cl.4) |
| A | US-A-4 203 442 (A.S. MICHAELS)<br><br>* Colonne 3, ligne 54 - colonne 4, ligne 29; colonne 5, lignes 37-48 *<br>--- | 1,10, 36,40 | |
| A | FR-A-2 331 320 (GENERAL ATOMIC COMPANY)<br>* Page 7, lignes 31-34 *<br>--- -/- | 1,25, 27 | |

Le présent rapport de recherche a été établi pour toutes les revendications

| Lieu de la recherche<br>LA HAYE | Date d'achèvement de la recherche<br>18-03-1985 | Examinateur<br>WOLF C.H.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen
des brevets

**RAPPORT DE RECHERCHE EUROPEENNE**

**0147274**
Numéro de la demande

EP 84 40 2445

| | **DOCUMENTS CONSIDERES COMME PERTINENTS** | | Page 4 |
|---|---|---|---|

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 040 417 (J.A. ZIPPER) <br><br> * Colonne 2, lignes 4-28 * <br> --- | 10,33, 36,39, 40,43 | |
| A | GB-A-2 024 020 (MEDHOLDING CY.) <br><br> * Figure 4 * <br> --- | 16,17, 20,21 | |
| A | CH-A- 568 069 (A.H. ROBINS COMPANY) <br> * Figures 18,19 * <br> --- | 16-21 | |
| A | US-A-3 407 806 (J.F. HULKA et al.) <br> * Colonne 3, lignes 17-20; figure 6 * <br> --- | 16,18, 19 | |
| Y | FR-A-1 500 461 (SOCIETE LE CARBONE-1ORRAINE) <br> * Page 2, colonne 1, ligne 6 - colonne 2, ligne 18 * <br> --- <br><br> ----- | 6,7,30, 34 | **DOMAINES TECHNIQUES RECHERCHES (Int. Cl.4)** |

Le présent rapport de recherche a été établi pour toutes les revendications

| Lieu de la recherche LA HAYE | Date d'achèvement de la recherche 18-03-1985 | Examinateur WOLF C.H.S. |
|---|---|---|